# EUROPEAN PATENT SPECIFICATION

(11) **EP 1 945 276 B1**
(45) Date of publication and mention of the grant of the patent: **23.06.2010**
(21) Application number: 06794704.4
(22) Date of filing: 10.10.2006
(51) Int. Cl.: A61L 2/24, A61L 2/18, A61B 1/12

(54) **ENDOSCOPE REPROCESSING APPARATUS**
ENDOSKOP-AUFBEREITUNGSGERÄT
APPAREIL DE RETRAITEMENT

(30) Priority: 10.10.2005 GB 0520551
(43) Date of publication of application: 23.07.2008
(73) Proprietor: PuriCore, Inc., Malvern PA 19355 (US)
(72) Inventor: OLIVER, Geoff, North Somerset BS21 6LH (GB); HOOPER, James, North Somerset BS21 6LH (GB)
(74) Representative: Brewster, Andrea Ruth
(86) International application number: PCT/GB2006/003753
(87) International publication number: WO 2007/042787

(56) References cited:
- US-A- 3 893 843
- US-A- 4 064 886
- US-A- 4 447 399
- US-A- 4 489 741
- US-A- 4 763 678

## Description

The present application relates to apparatuses and methods for the reprocessing of devices such as medical equipment, in particular endoscopes.

Endoscopes are devices used in medical investigations for examining and/or treating the interior of a bodily canal or hollow organ (such as the colon, bladder or stomach). Endoscopes typically have an external tube or casing containing a series of inner tubes, which serve various functions. For instance, the inner tubes may comprise flexible optical fibres which allow transmission of images to an optical system or miniature video camera. Other tubes may be used to transport liquids or gases, or to obtain biopsy samples. Examples of endoscopes include but are not limited to gastroscopes (also known as gastrointestinal endoscopes), auriscopes, bronchoscopes, nasopharygoscopes, fibroscopes, video-endoscopes and echoendoscopes.

Endoscopes are a valuable diagnostic and therapeutic tool in modern medicine, but they are typically complicated and expensive devices and not disposed after each use. Because they are exposed both internally and externally to bodily fluids and tissues, they need to be washed thoroughly and disinfected (or "reprocessed") before re-use. Many endoscopes such as flexible endoscopes are difficult to reprocess and may be easily damaged due to their intricate design and delicate materials. Reprocessing can include the steps of: (1) mechanical cleaning of internal and external surfaces, for example involving brushing and flushing internal channels with water and a detergent; (2) disinfection by immersion or spraying of the endoscope in a high-level disinfectant; (3) rinsing with sterile or filtered water; and (4) drying, for example with alcohol followed by dry air.

Endoscopes can be reprocessed by manual washing and sterilising, or using automated endoscope reprocessors (AERs, also referred to in the European standard as "washer disinfectors"). AERs offer several advantages over manual reprocessing, including automation and standardisation of key reprocessing steps, thereby avoiding the likelihood that a step is missed, and a reduction in the exposure of personnel to contaminants, high-level disinfectants or chemical sterilants. Mechanical cleaning followed by further reprocessing in an AER has been used in the prior art to attempt to achieve effective reprocessing (see Alvarado & Reichelderfer, 2000, AJIC Am. J. Infect. Control 28: 138-155).

Conventional AERs comprise a basin into which an endoscope is immersed. Nozzles or injectors in the basin which emit washing fluid, disinfecting fluid, rinsing fluid and/or drying air. Fluids are thereby intended to pass through the internal channels and outer surface of the endoscope. US5,494,637 describes an AER comprising a basin for holding an endoscope, fluid holding means coupled to the basin means for collecting reprocessing fluids, and a spray means such as nozzles coupled to the fluid holding means for simultaneously spraying an endoscope to be reprocessed, the basin means and the fluid holding means with a reprocessing fluid. US5,882,589 describes an AER where an endoscope to be reprocessed is immersed in a substantially tear-shaped basin where the external surface of the endoscope is washed and sterilised by cleaning fluid emitted from injectors arranged within the basin. The basin also contains couplings which attach to the endoscope to allow internal channels of the endoscope to be reprocessed. In US6,919,057, an AER is disclosed with a removable rack for receiving a container having two mating portions defining an internal pressurisable chamber into which an endoscope head can be placed for reprocessing. EP1253951 teaches an AER with a cabinet having spray nozzles for spraying a washing fluid and a microbial decontaminant fluid over an external surface of the endoscope. The AER further comprises a connector for connecting connection ports within the chamber to internal channels of the endoscope, allowing the reprocessing fluids to be passed through the endoscope. EP1253951 also discloses that the endoscope may be supported on a support member which can be agitated to change points of contact with the endoscope.

US 4,064,886 is an apparatus for cleansing endoscopes in which an endoscope is suspending from a holding device into a cylindrical cleaning container. The holding device rotates the endoscope about its axis, whilst it is sprayed by nozzles in the cylindrical cleaning container.

US 3,0893,843 discloses a method for decontaminating flexible articles, in which the articles are placed within a retaining basket which is rotatably mounted in a working tub. The retaining basket is designed to receive particular types of hollow articles and hold them in specific positions which will cause the liquid to circulate through them during agitation and empty them when the basket is spun.

US 4,763,678 is a cleansing system in which the control portion of an endoscope is held on an external surface of the apparatus whilst the insertion portion has been inserted into an insertion tank. The control portion is connected by small tubes to a cleaning system to permit cleaning fluid to flow through the endoscope's internal passageways. The insertion tank contains a rotary mounted container which holds the main body of the endoscope.

US 4,489,741 discloses an apparatus for washing an endoscope. An endoscope is inserted into a washing tank and upper and lower nozzles rotate, spraying washing liquid onto the endoscope. Inner passages of the endoscope are washed by supplying the washing liquid through tubes connected to the passageways.

US 4,447,399 discloses a combination steam and unsaturated chemical vapour sterilizer.

Following reprocessing, endoscopes should be stored in such a way as to prevent recontamination prior to re-use. For example, storage may involve hanging the endoscopes in a drying chamber through which sterile air is passed.

Despite the development of different types of AER, more healthcare associated outbreaks have been linked to contaminated endoscopes than to any other medical device (Spach et al., 1993, Ann. Intern. Med. 118: 117-128; Weber & Rutala, 2001, Infect. Control Hosp. Epidemiol. 22: 403-408; Rutala et al., 2002, Healthcare Infection Control Practices Advisory Committee's "Draft Guideline for Disinfection and Sterilization in Healthcare Facilities", available at http://www.myendosite.com/my_articles.htm). Major problems include inadequate reprocessing due to parts of the endoscope not be exposed to the reprocessing process because they are occluded surfaces closed off by valves or seals and/or because they are difficult to reach (for example intricate areas such as hinge joints or recessed surfaces). The present invention provides an improved apparatus and method suitable for endoscope reprocessing.

According to a first aspect of the invention there is provided an endoscope reprocessing apparatus (1) according to claim 1.

The present invention allows rotation of an endoscope relative to the reprocessing chamber so that exposure of the endoscope to reprocessing fluids is maximised. Rotation allows more effective cleaning, disinfecting and rinsing of the endoscope, particularly parts of the endoscope which are difficult to reach and/or occluded. The apparatus will allow reprocessing to be fully automated, with no mechanical pre-cleaning or pre-washing required apart from normal brushing through of channels to remove tenacious debris where necessary. Rotation may also facilitate drying of the endoscope, for example in that any liquids remaining on or in the endoscope can be encouraged to drain to one or more ends of the endoscope with assistance of gravity by angling the endoscope appropriately.

Although the apparatus in one aspect is suitable for reprocessing endoscopes, the apparatus may also be used for reprocessing other devices for example medical devices and/or devices more generally having a tubular member with a channel (such as a catheter).

The first reprocessing means is arranged to allow reprocessing fluid emitted therefrom to reprocess an exterior surface of the endoscope. Additionally, the reprocessing chamber may comprise a connector in fluid communication with the reprocessing fluid source, the connector attachable to an end of the endoscope to allow reprocessing fluid to contact an internal channel of the endoscope. In this way, the exterior and interior surfaces may be reprocessed by the apparatus (simultaneously or sequentially). Endoscopes may contain several internal channels, each of which may be contacted by the reprocessing fluid, separately or in groups.

The connector is coterminous with the mounting. An end of the endoscope is attached to the connector directly upon attachment of the endoscope carrier to the mounting. Alternatively, the connector may be attached to an end of the endoscope following attachment of the endoscope carrier to the mounting.

The reprocessing fluid source in fluid communication with the connector may be independent from, or operably linked with, the reprocessing fluid source in fluid communication with the first reprocessing means. When the reprocessing fluid sources are operably linked, the same reprocessing fluid contacts the exterior and interior of the endoscope.

The first reprocessing means may comprise a spray system, for example spray nozzles or spray injectors or spray bars with nozzles and/or injectors. The first reprocessing means, for example the spray system, may be fixed or rotatable within the reprocessing chamber. Where the first reprocessing means is rotatable within the reprocessing chamber, rotation of the endoscope carrier relative to the reprocessing chamber may be at a different angle or stage compared with rotation of the first reprocessing means. The endoscope carrier may in addition be rotated relative to the rotating shaft. Relative rotation may be optimised to allow reprocessing fluid to contact the endoscope positioned on or in the endoscope carrier at multiple and varying angles.

The first reprocessing means may be reversibly detachable. This allows removal, sterilisation (for example by autoclaving) and replacement of the first reprocessing means.

The mounting of the apparatus may engage with a rotary shaft disposed within the reprocessing chamber. In one aspect of the invention, the rotary shaft may be generally horizontally disposed within the reprocessing chamber. Alternatively, the rotary shaft may be generally vertically disposed or disposed at an angle within the reprocessing chamber.

The rotary shaft may be operably connected to a drive means which rotates the endoscope carrier relative to the reprocessing chamber. Additionally or alternatively, the endoscope carrier may be rotated relative to the reprocessing chamber by reprocessing fluid emitted from the first reprocessing means.

The endoscope carrier may be rotated relative to the reprocessing chamber through up to 360 degrees, for example up to 180 degrees. In this embodiment, the endoscope carrier oscillates (i.e. rotates back and forth, rather than rotating in one direction). Rotation during one or more of the various reprocessing steps as described herein may be varied to optimise reprocessing. For example, rotation may be in one direction during one reprocessing step and oscillatory during another reprocessing step.

The endoscope processor may have one or a plurality of openable doors and/or openable windows through which the endoscope carrier and/or the endoscope is removably attachable. Rotation of the endoscope carrier allows one or more endoscopes to be positioned on or in the endoscope carrier (or loaded) at one side of the apparatus and removed from the endoscope carrier at another side of the apparatus. Similarly, rotation of the rotary shaft allows one or more endoscope carriers to be attached (or loaded) on the mounting at one side of the apparatus and removed at another side of the apparatus. In this way, loading of used endoscope(s) and/or endoscope carrier(s) may for example take place in a designated contaminated area while removal of the reprocessed endoscope(s) and/or endoscope carrier(s) may take place in a designated clean area.

In one embodiment, the endoscope carrier remains substantially stationary while the first reprocessing means is rotated relative to the carrier.

The reprocessing fluid source may comprise one or a plurality of receptacles. The receptacles may be storage receptacles such as disposable containers which contain reprocessing fluids (for example, concentrated chemicals).

The reprocessing fluid source may additionally or alternatively comprise one or a plurality of reprocessing fluid reservoirs (or tanks). The reprocessing fluid reservoir(s) may be used for the storage, preparation, disposal and/or transfer of reprocessing fluid. The reprocessing fluid reservoir(s) may be in fluid communication with the above-mentioned receptacle(s), for example such that reprocessing fluid is passed from the receptacle(s) to the reservoir(s) en route to the reprocessing chamber. The reprocessing fluid reservoir may receive water from a purification filter (for example an ultrafiltration filter with typically a 0.04 micron filter) or a reverse osmosis unit either or both of which may be an integral part of the apparatus.

The reprocessing fluid source may be operably connected with a delivery system which delivers reprocessing fluid (for example from the reprocessing fluid reservoir[s]) into the reprocessing chamber.

In one embodiment, the delivery system comprises a pump. Where the apparatus comprises a plurality of reprocessing fluid reservoirs, the delivery system may be operably connected with a single reservoir.

The reprocessing chamber may comprise an outlet for drainage of reprocessing fluid. The outlet may be in fluid communication with the reprocessing fluid source (for example the reprocessing fluid reservoir[s] and optionally also with the receptacle[s]).

According to a further aspect of the invention the apparatus comprises a rotating drain valve. The rotating drain valve may be in fluid communication with the outlet of the apparatus. The rotating drain valve may comprise an indexing conduit rotatable to align with one of a plurality of exit conduits in fluid communication with the reprocessing fluid source (for example, the reprocessing fluid reservoir[s] and/or the reprocessing fluid receptacle[s]) and/or a gas extractor.

The apparatus may comprise a gas inlet in fluid communication with the reprocessing chamber, for example in which the gas inlet comprises a HEPA filter.

The rotating drain valve may be operably linked to a drive mechanism for rotation of the indexing conduit to align with one of the plurality of exit conduits.

The apparatus in one aspect allows the delivery and return of reprocessing fluids (for example, different grades of water and different chemicals to be sprayed onto endoscopes) using a number of reprocessing fluid reservoirs (or tanks), where each reprocessing fluid receptacle may be independently assigned to carry a different reprocessing fluid (for example, different grade of water or different chemical) in any combination. Different reprocessing fluids (for example, different grades of water or different chemicals) can then be taken from any one of the reprocessing fluid reservoirs and delivered under pressure by a common delivery system to the (optionally sealed) reprocessing chamber in which endoscopes may for example be sprayed. At the same time, other reprocessing fluids (for example, other grades of water or chemicals) can be prepared in idle reprocessing fluid reservoirs. Any number of reprocessing fluids (for example, grades of water or chemicals) may be prepared in some reprocessing fluid reservoirs while other reprocessing fluid reservoirs are in service. Activities in idle reprocessing fluid reservoirs might include the preparation of detergents, disinfectants or purified water and the heating of chemicals (for which see below).

After being emitted into the reprocessing chamber, for example by spraying, different reprocessing fluids (for example, grades of water and chemicals) may be returned under gravity to a common outlet and then diverted either to the reprocessing fluid reservoir that was an original source or the reprocessing fluid, or other reprocessing fluid reservoirs (or receptacle[s]) or external locations, in any combination. An advantage of using multiple reprocessing fluid reservoirs is that while one or more of the reprocessing fluid reservoirs are in service, other reprocessing fluids (for example, water and chemicals) can be prepared in idle reprocessing fluid reservoirs without needing to stop the reprocessing (for example, spraying) operation. This means that the reprocessing process (for example, cleaning, disinfecting and rinsing operations) may progress unhindered, enabling the cycle time of the apparatus to be kept to a minimum.

Therefore, in operation, the reprocessing fluid may be delivered to the reprocessing chamber from a single reprocessing fluid reservoir (at any given time). Where the apparatus comprises a plurality of reservoirs, the second or further reservoirs may then be used to prepare further reprocessing fluid, for example after rinsing of the reservoirs. The time constraints for reprocessing an endoscope is therefore determined by the required contact time between the -reprocessing fluid and the endoscope as a reprocessing cycle can continue simultaneously with the preparation of reprocessing fluid for a subsequent reprocessing cycle.

The present invention allows the same reservoir (or tank) to be used for different reprocessing fluids. An advantage of this is that there are benefits during a cleaning and disinfection cycle of being able to charge the reservoirs with different grades of water. In the early stages of a reprocessing cycle, there may be a need for using filtered water to prepare some chemicals while later in a cycle purer water may be needed. At different stages in a cycle there are also benefits in being able to use different grades of rinse water. At the beginning of a cycle, it is acceptable to use filtered water whereas at the end of a cycle very pure water is needed. Reallocation of the purpose of each reservoir with the same number of reservoirs needed to receive different chemicals allows preparation of different grades of pure water. It is then possible to use water in each reservoir progressively to achieve increasingly clean rinses. For example, one reservoir may be put on line (i.e. used for transmitting reprocessing fluid into the reprocessing chamber) to achieve an initial rinse and the water it contains drained away before a second reservoir with pure water is put on line.
Because the system drains down completely each time a new reservoir comes on line, there is less contamination to remove following each rinse cycle and highly efficient rinsing can be achieved. Two common grades of water that are used include filtered tap water and reverse osmosis water. A further advantage of being able to swap different fluids in each reservoir is that some chemicals may need to be heated and a common reservoir or reservoirs that can be heated can be used to heat different reprocessing fluids (for example, different grades of water or different chemicals).

The rotating drain valve of the present invention may rely on gravity to empty the reprocessing chamber until virtually no residue remains. This ensures that a single return system can be used to handle any number of reprocessing fluids with virtually no cross contamination. In addition, since all fluids are returned under gravity, in the event of any failure of the apparatus (for example, due to a power failure), reprocessing fluids can be returned to a reservoir where they can be contained safely. To ensure that the reprocessing chamber is connected only to a reservoir (or tank) which is "on line", the rotating drain valve is designed to achieve an air-tight seal between the "live" reservoir and the reprocessing chamber.

The reprocessing chamber of the present invention may be pressurisable and/or depressurisable (i.e. able to form a vacuum or partial vacuum). The pressure or vacuum may be used to inhibit or remove foam that can be generated by certain reprocessing fluids (for example, certain chemicals).

An additional feature of the rotating drain valve is that its common source may be a single outlet (leading to the indexing conduit) in the base of the sealed reprocessing chamber. In addition to receiving different grades of reprocessing fluid (such as water and chemicals) from the reprocessing chamber, the same valve can also be used in reverse to connect the reprocessing chamber to a gas source (for example, clean air) for fume extraction. By connecting a valve exit conduit to a gas duct (for example, an air duct), fumes in the reprocessing fluid reservoir(s) and the valve may be continuously extracted by directly connecting these to the gas duct. Fumes in the reprocessing chamber may be removed just prior to the reprocessing chamber being opened (for example, for removal of a reprocessed endoscope or endoscope carrier) by using the valve to connect the outlet of the reprocessing chamber to the gas duct. Extract air may for example then be drawn from the top of the reprocessing chamber through a chemical filter.

An advantage of the above embodiment is that internal air spaces in reprocessing fluid reservoir(s), the rotating drain valve and the reprocessing chamber can be ventilated using clean filtered air or other gas from a common source. Using clean air prevents introduction of microbial contamination in the air spaces of the delivery and return systems and/or endoscopes. Using clean air to ventilate the reprocessing chamber at the end of a reprocessing cycle also means that the chamber will contain clean air prior to being opened, for example on a clean side. In addition, if the gas (for example, single air source) is heated, warm gas passing through the reprocessing chamber can be used to dry the exterior and optionally interior surfaces of reprocessed endoscopes.

In one embodiment, the reprocessing chamber comprises one or more fluid vents which are reversibly openable to allow reprocessing fluid to enter and/or exit the chamber. For example, the reprocessing chamber may comprise an air vent which allows draining of reprocessing fluids from the reprocessing chamber. Alternatively, the vent may be used to pass fluid such as compressed gas or air through the reprocessing chamber. Passing compressed air or other gas through the channels of an endoscope positioned on or in the endoscope carrier improves drying of the endoscope. Such vents may be additional to the rotating drain valve described herein. The vent or vents may be connected to the common source in fluid communication with rotating drain valve. Fluid such as compressed gas or air may be filtered or otherwise sterilised as described above. The reprocessing chamber may in this way allow reprocessing to proceed in a clean air environment.

The vent(s) and/or rotating drain valve may be used to generate the pressure or vacuum under which the reprocessing chamber may operate.

According to a further aspect of the invention, the endoscope carrier may comprise a rack.

Alternatively, the endoscope carrier may be a cartridge (which term is used synonymously with "cassette"). The cartridge may comprise a second reprocessing means disposed on or in an internal surface of the cartridge and in fluid communication with the reprocessing fluid source. The reprocessing fluid source in fluid communication with the cartridge may be independent from or operably linked with the reprocessing fluid source in fluid communication with the first reprocessing means and/or the connector. The reprocessing fluid emitted from the first and second reprocessing means may be from the same reprocessing fluid source, so that fluid entering the cartridge is the same as fluid entering the processing chamber.

The second reprocessing means may emit reprocessing fluid which contacts an exterior surface of an endoscope housed within the cartridge, while the reprocessing fluid emitted from the first reprocessing means contacts an exterior surface of the cartridge (rather than the endoscope itself). In this way, the exterior of the cartridge and its contents are subjected to reprocessing fluid and are thereby, for example, cleaned and disinfected.

The cartridge may engage with the connector as defined above to allow reprocessing fluid to contact a channel of an endoscope housed within the cartridge and/or to provide means for fluid communication between the reprocessing fluid source and the second reprocessing means.

The cartridge may have a latching device (for example a bayonet fitting) to allow connection with the connector of the apparatus.

The endoscope carrier (including the cartridge), for example prior to insertion of an endoscope, may be autoclavable. The reprocessing chamber may be removably detachable and optionally also autoclavable (or treatable with chemical disinfectants).

The cartridge and/or rack may be transferable between the reprocessing chamber and a storage chamber, for example a storage chamber into which the cartridge and/or rack is connectable to an air supply for sterile and/or dry storage of a processed endoscope within the cartridge. The cartridge and/or rack in this way allows the processed endoscope to be stored without separate reconnection of the endoscope ends to and air supply, limiting handling of the processed endoscope *per se* prior to re-use.

The endoscope carrier may be shaped to receive one or more endoscopes. For example, the cartridge and/or rack may comprise indentations to receive one or more components of the endoscope. The endoscopes may fit loosely within the endoscope carrier to allow contact points between the endoscope and endoscope carrier to change during rotation of the endoscope carrier, thereby allowing all points of the exterior of the endoscope to be contacted with the reprocessing fluid.

Endoscopes of any size may be mounted in an endoscope carrier, such as a rack (or frame, for example, a portable wire holding rack) or a cartridge, that is tailored to suit the shape of different types of endoscope.

The endoscope carrier may comprise a receptacle that attaches to a spigot located on or operably linked with the mounting or the reprocessing chamber. Purposes of the receptacle and spigot may include providing a means of support and a method of making multiple connections between internal channels of an endoscope and the reprocessing fluid (or irrigation system) of the apparatus. The spigot can also be shaped or keyed to align holding frames or cartridges in the reprocessing chamber.

The endoscope carrier may be portable. Endoscopes may be prepared and/or positioned on or in the endoscope carrier away form the apparatus, transported to the apparatus when ready and following reprocessing, removed and transferred in their holding frame or cartridge to a storage cabinet. The storage cabinet can then be designed with spigots or mountings functionally identical or similar to those in the reprocessing chamber, so that on being plugged in, the internal channels of an endoscope can be connected to a clean air purge system without disturbing the endoscope.

A similar mounting on a wall-mounted or other unit may be used to store an empty endoscope carrier or a cartridge containing contaminated endoscopes that are awaiting reprocessing.

The use of a cartridge to keep an endoscope enclosed in a separate environment during reprocessing and storage has not previously been described and is desirable for safer and cleaner handling of endoscopes.

It is possible to use the spigot described above to enable additional fluid connections to the second reprocessing means (for example, an internal spray system). The second reprocessing means may then be housed inside and remain an integral part of the cartridge, enabling the cartridge to remain portable. Where the second reprocessing means is a spray system, this can be fixed or rotating and in either case the spray system and cartridge may be aligned with each other using the spigot. In the case of a rotating system, for example, part of the spigot may be used as the pivot on which a spray system can rotate while the remainder of the spigot holds the cartridge in alignment with the spray system. This will enable the spray system to rotate freely without interference from fixed parts of the cartridge. If a rotating spray system is used, the spray system may be captive in the cartridge so that it travels with the cartridge on being removed to other locations. If a fixed spray system is adopted, the walls of the cartridge can wrap over the spigot to enable galleries in the walls of the cartridge to align with fluid connections in the spigot. In a rotating system spray nozzles may be located around a central cylinder and spray bars may be mounted off the internal cylinder.

When a rotating spray system is used, rotation can either be achieved by passing a driveshaft through the spigot so that this engages with the internal spray system or by relying on reaction forces due to spraying. The drive shaft can be connected to an external drive system, which may be operably linked to the drive means of the reprocessing chamber. An extension of the spigot may be used to connect the internal channels of an endoscope within a cartridge to an irrigation system as described above. By making the spray system an integral part of the cartridge, the cartridge remains portable. In certain countries, current codes of practice require that each channel of endoscope is independently irrigated. The present invention allows the cartridge to be attached to the apparatus with a single connection in fluid communication with each of the channels (for example, up to seven channels) in addition to the cartridge spray system.

The apparatus may comprise a heater and/or cooler to heat and/or cool the reprocessing fluid and/or the reprocessing chamber. For example, the reprocessing fluid may be heated in the reservoir(s) prior to entry into the processing chamber.

The reprocessing fluid comprises any one or more of the group consisting of water (for example purified water, pure water and/or reverse osmosis water), disinfectant (or sterilant; for example peracetic acid, a peroxygen compound, chlorine dioxide and/or 2% activated glutaraldehyde) and air.

In one embodiment, the apparatus is an automated endoscope reprocessor (AER).

The apparatus may further comprise a control means for controlling, the supply, recirculation and/or discharge of reprocessing and/or other fluids. The control means may control the various reprocessing steps for example as described herein including rotation of the endoscope carrier.

Reprocessing as used herein typically involves one or more or all of the steps of cleaning, disinfecting, rinsing and drying.

Reprocessing may be effected by chemical and/or mechanical and/or thermal means. For example, chemical means may include an initial washing of the endoscope using a detergent and decontamination (washing) solution to eliminate organic matter. This removes any potential support for germs by solubilising proteins and saponifying fatty matter. After rinsing with a rising solution (for example, purified and/or sterile water), the endoscope may be disinfected in a disinfecting solution, such as a chlorine- or peracetic acid-based solution providing bactericidal, virucidal, algaecidal, fungicidal and/or sporicidal activity. The washing, rinsing and/or disinfecting solutions may be hot. Mechanical reprocessing may be effected by the reprocessing means, for example the spray system. Due to rotation of the endoscope carrier and/or the reprocessing means, the reprocessing fluid emitted from the reprocessing means contacts the endoscope at different angles, thereby eliminating sedimentation, clogging or obstruction. The reprocessing fluid may be emitted at pressure (for example 0.5 bar to 4 bar). Thermal reprocessing may allow optimisation of the chemical and/or mechanical means, for example by maintaining the reprocessing fluid at a temperature of somewhere between about 25°C and about 60°C (or to about 55°C for thermolabile endoscopes) where this is suitable for the type of endoscope being reprocessed.

Also provided according to the present invention is a method for reprocessing an endoscope, comprising the step of subjecting the endoscope to a reprocessing cycle within an endoscope reprocessing apparatus as described herein. The reprocessing cycle may comprise one or more or all of the reprocessing steps as described herein.

In further aspects of the present invention there is provided: a reprocessing chamber as described herein for an endoscope reprocessing apparatus; a rotating drain valve as described herein for an endoscope reprocessing apparatus; and an endoscope cartridge as described herein for reprocessing and optionally storing one or a plurality of endoscopes.

It is normal practice when irrigating the channels in an endoscope to inject fluid under pressure using the connections on an endoscope to pass liquid through channels towards the distal end (the end which enters the human body) of the endoscope. In the majority of cases, at least two channels combine into one before reaching the distal end. In some cases, as many as three channels can combine before reaching the distal end as a single channel. Fluid flow in each of these channels may be monitored to ensure that the correct amount of liquid is passing through each channel for reprocessing purposes.
Normally, flow and pressure sensors are located in the flow prior to entering connections on an endoscope so that the flow through each channel can be monitored. It is important to ensure that there is flow at the distal end but if there is a significant amount of leakage at any of the connections on an endoscope, the amount of fluid that actually reaches the distal end could be considerably reduced and this cannot be detected solely by monitoring inflow. It is therefore important to detect a pressure drop to determine the presence of a leak. Because operating pressures are often low, this is not always easy to determine at the point where fluid enters the connection on an endoscope because the difference in pressure between normal operation and operation with a leak is virtually the same. It is therefore desirable to have a monitoring technique whereby flow at the distal end can be deduced.

One solution is to measure flow out of the distal end but this requires a connection to the distal end. This is not ideal because it would be difficult to properly clean and disinfect the distal end. A novel way of overcoming these difficulties where flow in and out of the connections on an endoscope should be monitored has therefore been devised.

According to a further aspect of the invention, there is provided a method of monitoring the flow of reprocessing fluid through a channel of an endoscope connected to a reprocessing fluid source in an endoscope reprocessing apparatus (or AER), comprising the steps of drawing liquid in through a distal end of an endoscope and monitoring the flow that emerges at points of connections between the endoscope and the reprocessing fluid source.

In an alternative aspect there is provided a method of monitoring the flow of reprocessing fluid through a first channel of an endoscope connected to a reprocessing fluid source in an endoscope reprocessing apparatus (or AER), the endoscope having a second or further channels in fluid communication with the first channel, comprising the step of passing fluid under pressure through the first channel using the connection between the endoscope and the apparatus to admit reprocessing fluid while leaving open any further channels in fluid communication with the first channel. Whenever fluid is flowing through a channel, there is a continuous pressure drop throughout the length of that channel. At points where other channels join there is therefore a positive pressure with respect to atmosphere. The distal end and open channels will always be at atmosphere pressure and this means that there is always positive pressure with respect to atmospheric pressure at points where other channels meet a first channel. This means that fluid in a channel where there is positive pressure will backflow into open channels. Where a first channel is irrigated, it is possible to measure the amount of fluid flowing back through open (i.e. the second or further) channels. If the flow in open channels is correct it can be deduced that the amount flowing at the distal end is correct. If there is a leak in the first channel, the amount of fluid flowing towards the distal end will be reduced and likewise the amount of fluid that can be backfed through open channels will be reduced. This provides a clear indication of leakage and a positive way of monitoring whether or not there is an adequate fluid flow at the distal end of an endoscope. The method is not suitable for use with endoscopes that contain valves preventing a backflow.

Various aspects of the invention will now be described by way of example only with reference to the accompanying drawings in which:
Fig. 1 is a front view showing various internal components of an apparatus of the invention;
Fig. 2 is a rear view of the apparatus provided in Fig. 1;
Fig. 3 is a left-hand side view of the apparatus provided in Figs 1 and 2;
Fig. 4 is a right-hand side view of the apparatus provided in Figs 1-3;
Fig. 5 is an enlarged view of a reprocessing chamber from the apparatus provided in Figs 1-4;
Fig. 6 is a diagram showing sprayings steps in the apparatus provided in Figs 1-5;
Fig. 7 is an enlarged view of a rotary shaft and associated components of the reprocessing chamber from the apparatus provided in Figs 1-5; and

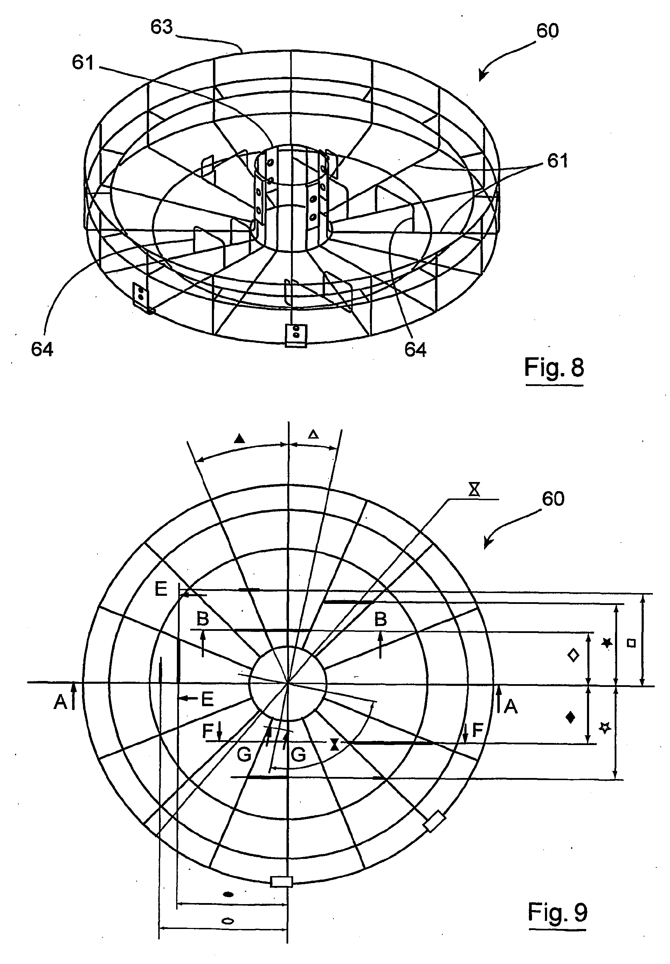

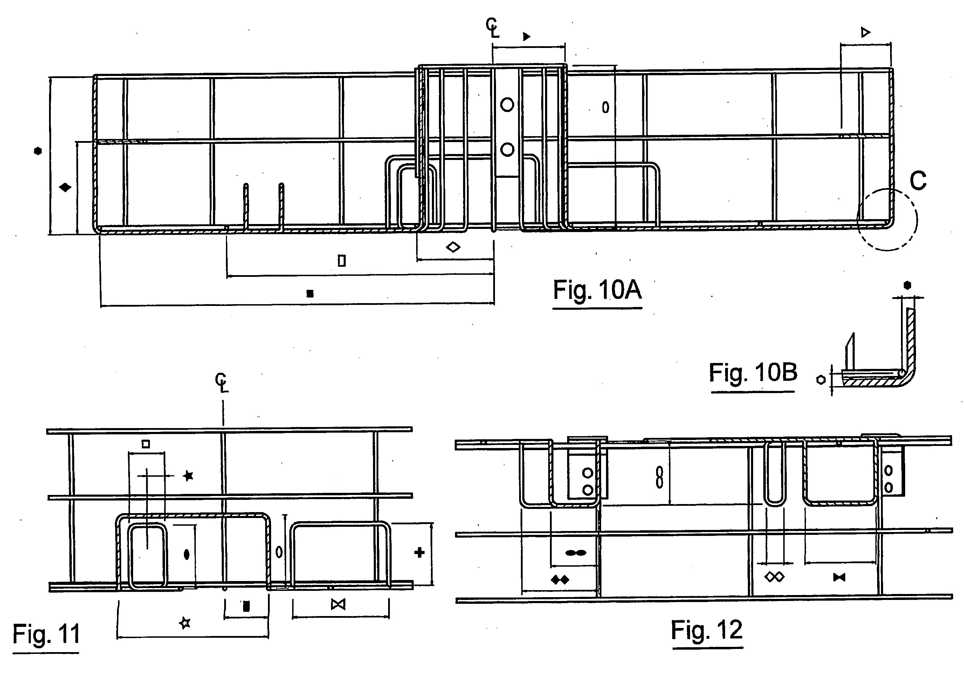

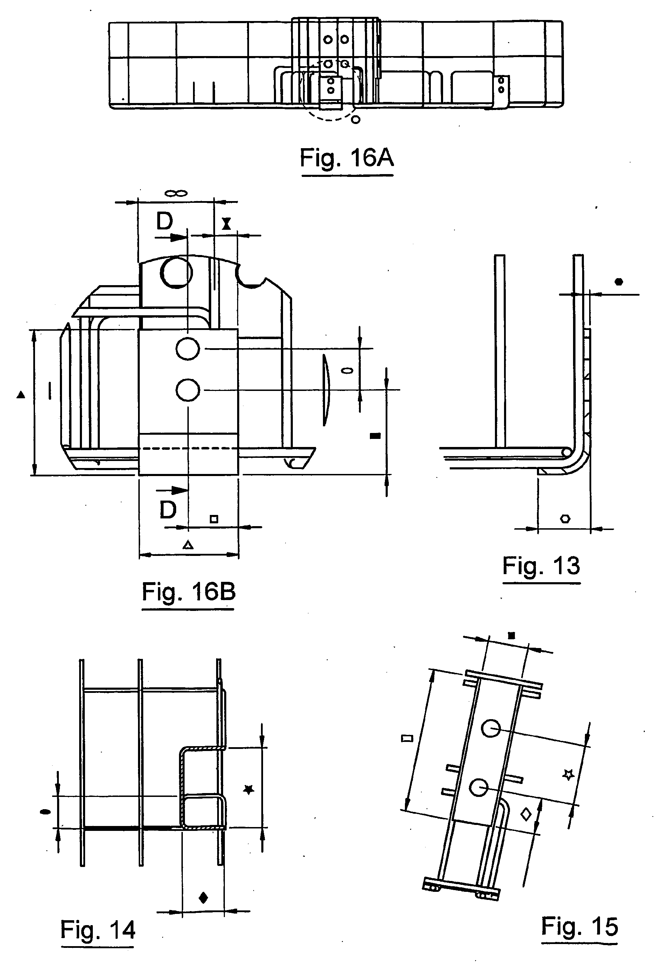

Figs 8-16 show in various views and sections an endoscope carrier according to a second embodiment of the invention. Fig. 8 provides an isometric view of the endoscope carrier, while Fig. 9 provides a top view of the endoscope carrier. Fig. 10A is a section through line A-A shown in Fig. 9, while Fig. 10B shows detail of the circled area C in Fig. 10A. Fig. 11 is a section through line B-B shown in Fig. 9. Fig. 12 is a section through line F-F shown in Fig. 9. Fig. 13 is a section through line D-D shown in Fig. 16B (see below). Fig. 14 is a section through line E-E shown in Fig. 9. Fig. 15 is a section through line G-G shown in Fig. 9. Fig. 16A is a cross-section view of the endoscope carrier, while Fig. 16B shows detail of the circled area O in Fig. 16A.

Symbols in the drawings denote as follows: in Fig. 9, ▲ = 22.5°(TYP 16 PLACES), △ = 11.25°, 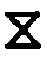 = ∅450, 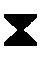 = 90°, ● = 120, ○ = 140, 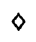 = 60, 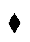 = 65, 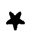 = 93, 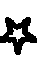 = 105, and 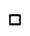 = 105; in Fig. 10A, 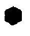 = 87, 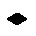 = 51, 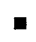 = (R222.4), 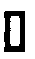 = R151. ◊ = R43.6, ► = R41, **0** = 90, and ▷ = 28.2 TYP; in Fig. 10B, 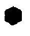 = 3.6, and 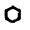 = 3.6; in Fig. 11, □ = 20, ★ = (10), 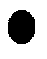 = 35, ☆ = (84.9), 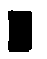 ★= (24.9), 0 = 40, 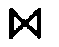 = (54.5), and **+** = 34; in Fig. 12, 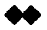 = (43.5), = (26.9), 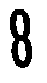 = 35, ◊◊ = 10, and 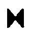 = (40); in Fig. 13, 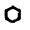 = 11.5, and 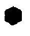 = 1.5; in Fig. 14, 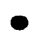 = 20, 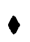 = 25 TYP, and 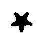 = (49.7); in Fig. 15, 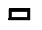 = 60, 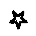 = 25, 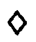 = 15, and 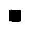 = 16; and in Fig. 16B, ▲ = 33.5, △ = 22, 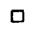 = 11, 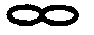 = 17, 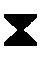 = 5, ○ = 9.5, and 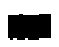 = 19.5.

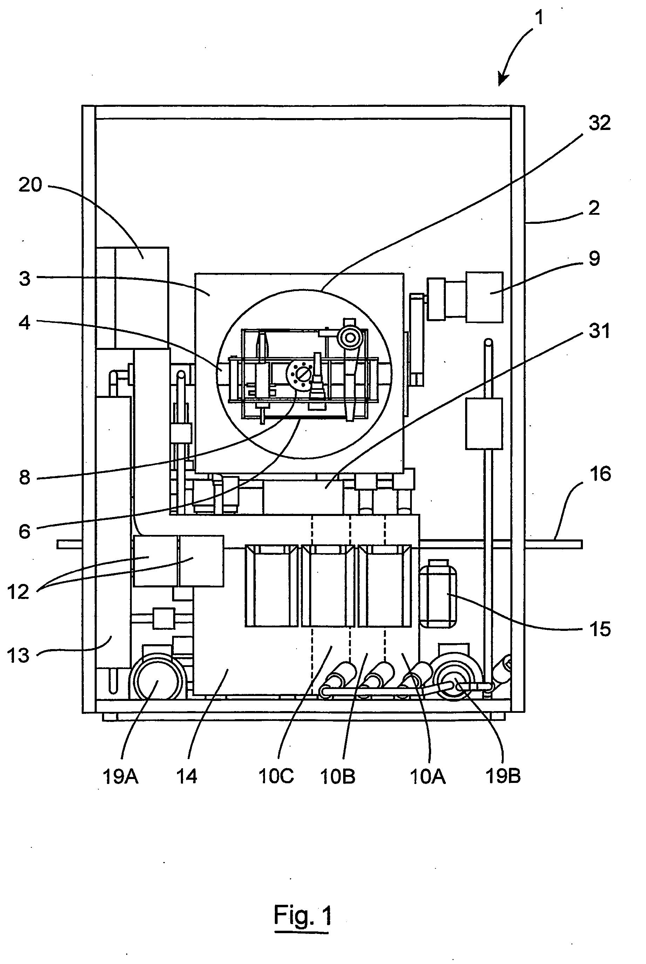

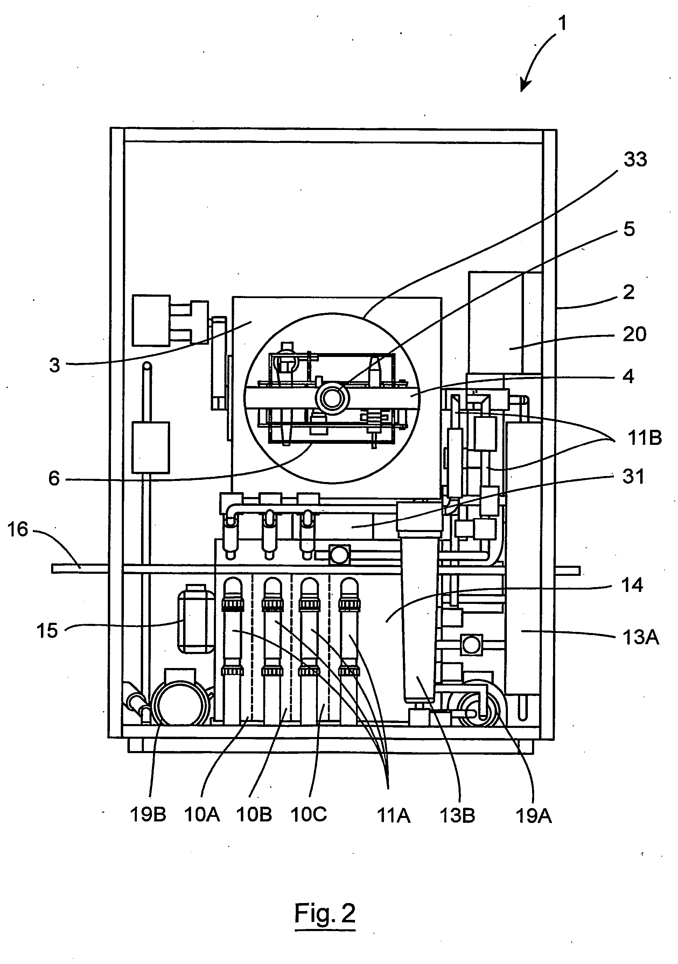

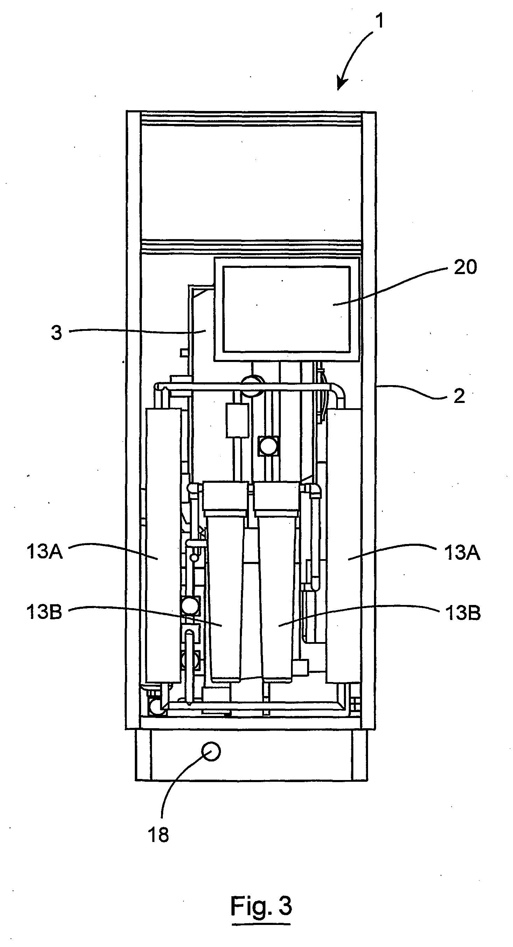

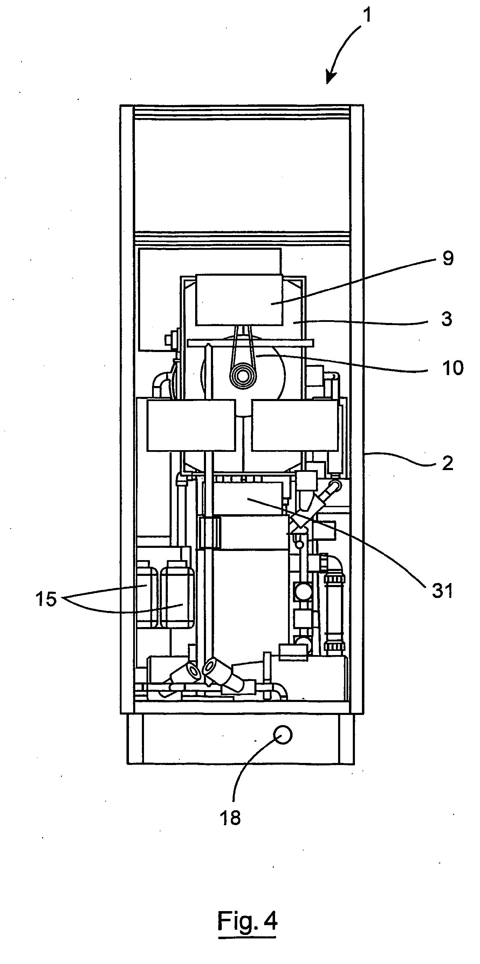

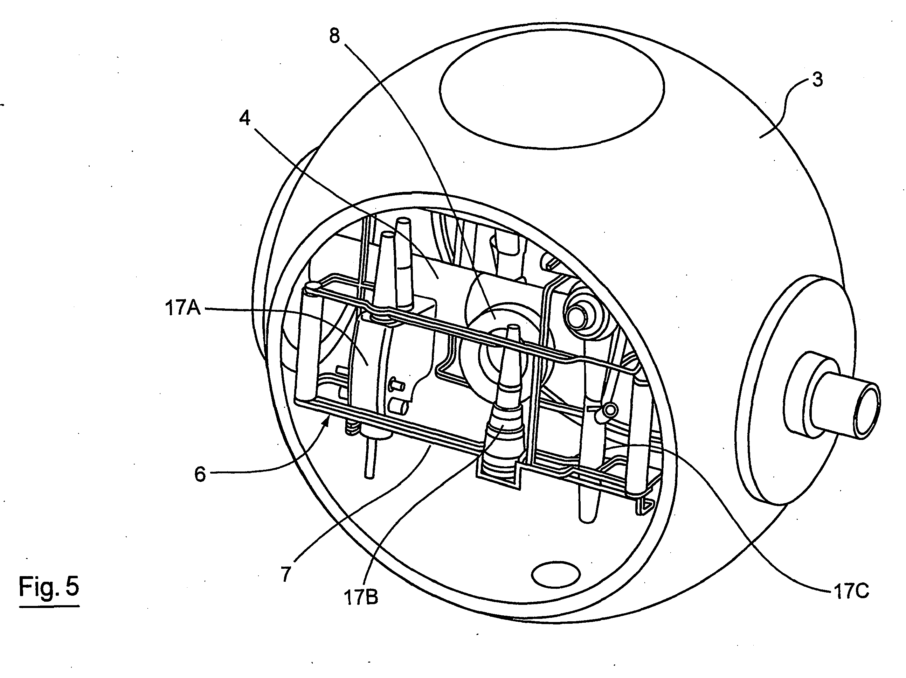

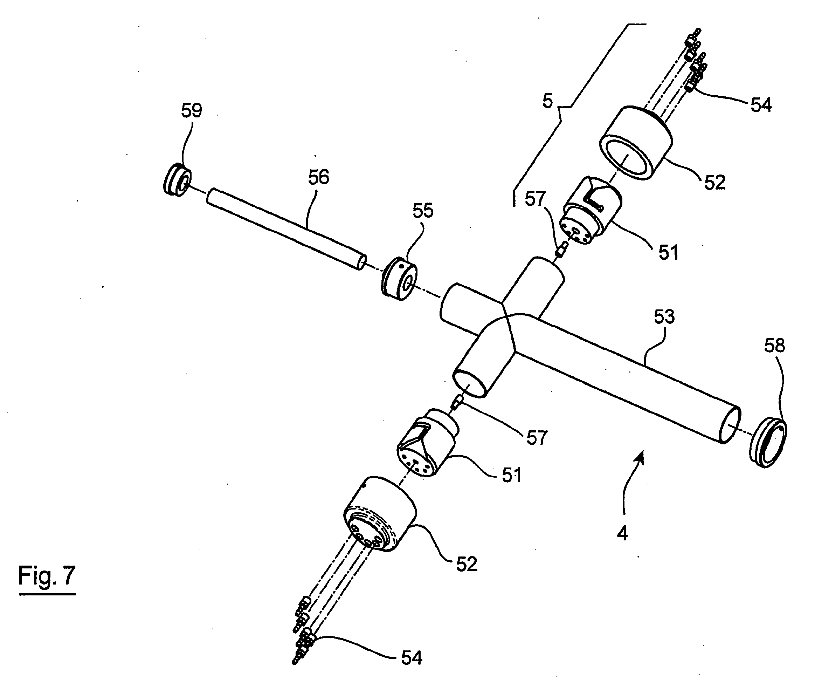

As depicted in Figs 1-4, an endoscope reprocessing apparatus 1 according to a first embodiment of the invention comprises an external housing 2 in which the various components of the apparatus are positioned. A reprocessing chamber 3, also shown in Fig. 5, is located within the housing and comprises two circular openable doors (see Fig. 7 as described below), one facing the front and the other the back of the apparatus (shown as circular openings 32, 33 in the reprocessing chamber 3 in Figs 1 and 2, respectively). Rotating spray bars (see Fig. 7 as described below) are located on a surface of each door facing into the reprocessing chamber. As can be seen in Figs 1, 2 and 5, a rotary shaft 4 is located centrally within the reprocessing chamber and comprises a first endoscope carrier mounting 5 (see Fig. 2), with a second endoscope carrier mounting (not visible) positioned opposite to the first endoscope carrier mounting.

In the embodiment shown in Figs 1-5, an endoscope carrier 6 comprising a hinged, openable and lockable shaped metal rack 7 is connected to a mounting of the rotary shaft via a receptacle 8. Endoscope heads 17A, 17B and 17C (but for clarity not flexible tubing and other parts of an endoscope) are shown in position within the endoscope carrier. The endoscope is loosely held by the metal rack of endoscope carrier. Tubing from the endoscope is connected via a receptacle on the endoscope carrier which fits into a spigot (not shown) located on each of the endoscope carrier mountings of the rotary shaft.

The rotary shaft is operably connected to a mechanical drive means comprising a drive belt 10. The rotary shaft is in fluid communication with reprocessing fluid reservoirs 10A-C via transfer pipes 11A,B. The reservoirs are in fluid communication with dosing chambers 12, water purifiers 13A,B (for example, reverse osmosis units 13A and filtered water tanks 13B with 5, 1 and 0.2 micron filters), a break tank 14 (a special version of a reprocessing fluid reservoir) and reprocessing fluid receptacles 15 which contain chemicals in an undiluted form. (In an alternative embodiment, a jump valve rather than a break tank is used.) Tap water enters the apparatus into the break tank by means of a water pipe 16. An outlet pipe 18 allows waste fluid to be removed from the apparatus. Reprocessing fluid is passed between the various containers via pumps 19A,B. The reservoirs and the chamber are also in fluid communication with an HEPA air inlet 20. Air exiting the apparatus from the reservoirs and chamber is passed through a carbon filter (not shown).

A first control computer (not shown) is operably linked to the mechanical and electrical components of the apparatus to control the reprocessing steps. A second separate and independent control computer (not shown) allows monitoring of a reprocessing cycle (for example, determining flow through an endoscope channel). The control computers are linked to a control panel (not shown) which allows a user of the apparatus to initiate, monitor and stop reprocessing.

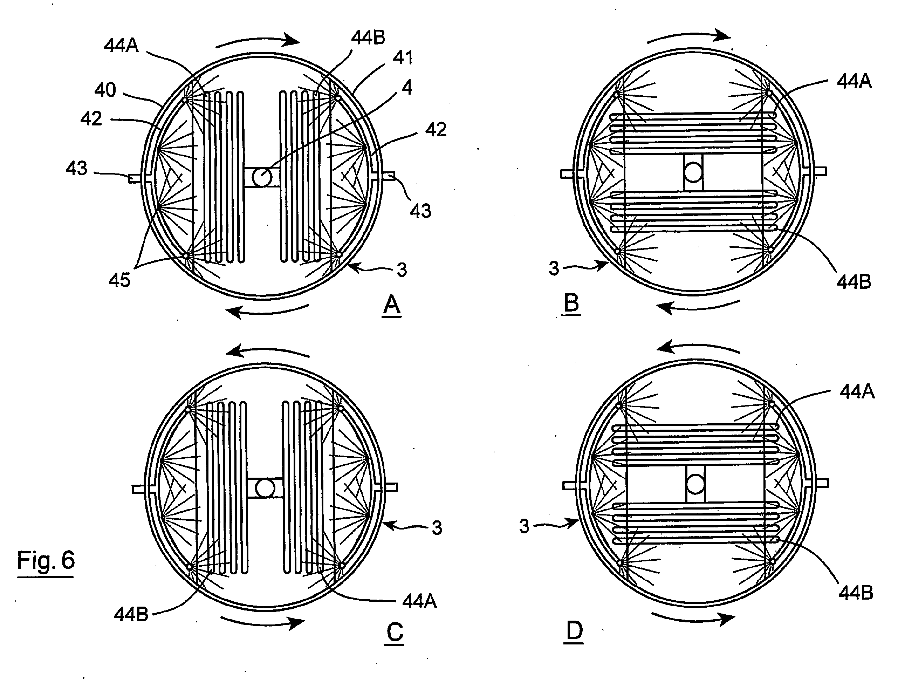

In operation, an endoscope carrier into which an endoscope has been position is connected to the first endoscope carrier mounting through the door facing the front of the apparatus, i.e. facing a dirty area. The rotary shaft is rotated through 180 degrees so that a second endoscope carrier can be connected to a second endoscope carrier mounting located opposite the first mounting. The door is closed, thereby sealing the reprocessing chamber, and a reprocessing cycle initiated to allow the endoscopes to be washed, disinfected, rinsed and dried. During this process, the endoscopes are rotated through 180 degrees and back, while the spray bars (for which see Fig. 6 below) are similarly rotated. After each reprocessing step, waste fluid from the reprocessing chamber is drained via a central outlet 30 (see Fig. 5) into a rotating drain valve 31, and then into an allocated reservoir or a main drain. The rotating drain valve in one embodiment forms a fluid-tight seal between the reprocessing chamber and the allocated reservoir or main drain. The drainage process is gravity-assisted. After the reprocessing cycle has been completed, the first endoscope is removed into a clean area at the rear of the apparatus through the door facing the back of the apparatus. The rotary shaft is turned through 180 degrees to allow removal of the second endoscope.

Spraying steps are illustrated in Fig. 6. The reprocessing chamber 3 has two doors, a door 40 opening at the front of the apparatus (i.e. at the dirty area side) and door 41 opening at the back of the apparatus (i.e. at the clean area side). Each door has a spinning shower bar 42 which is pivotally located on a shower bar pivot 43. Each spinning shower bar has nozzles 45 from which reprocessing fluid is sprayed. Two coiled endoscopes 44A, 44B are rotated on the endoscope carrier (not shown) around the rotary shaft 4 through the positions shown in Figs 6A to D, respectively, while the spinning shower bars are rotated in the direction shown by the arrows in Figs 6A-D.

Various components of the rotary shaft 4 and mounting 5 are shown in more detail in Fig. 7 and comprises two rotation spindle irrigation connectors 51, two endoscope carrier irrigation connectors 52, a large rotation spindle 53, ten 1/8 hosetails 54 for connecting 4 mm tubing of an endoscope, a spindle adaptor 55, a short rotation spindle 56, two irrigation connector tension spigots 57, a large rotary shaft bush 58 and a small rotary shaft bush 59.

An alternative endoscope carrier 60 for use in the apparatus as described above is provided in Figs 8 to 16. The endoscope carrier 60 comprises arms 61 extending radially from a central circular receptacle 62 to an outer perimeter 63. The endoscope carrier is connected to an endoscope carrier mounting of the rotary shaft (see Figs 1, 2 and 5) via the receptacle. Retainer bars 64 located at various positions on the arms are designed to hold an endoscope in position. The endoscope carrier has a fully welded construction, details of which are provided in Fig. 9 to 16. The exemplar endoscope carrier shown in Fig. 10A has a radius of about 222 mm and a maximum depth of about 90 mm. In use, endoscopes are positioned within the endoscope carrier (not shown). An endoscope carrier is connected to a rotary shaft mounting on each side of the rotary shaft, whereupon the outer perimeters of two connected and adjacent endoscope carriers form a contact seal.

## Claims

1. An endoscope reprocessing apparatus (1) comprising a reprocessing chamber (3), a mounting (5)to which an endoscope carrier (6) is attached, a first reprocessing means arranged on or in an internal surface of the reprocessing chamber, and a reprocessing fluid source (10A-C) in fluid communication with the first reprocessing means, a connector (52) in fluid communication with the reprocessing fluid source, the connector attachable to an end of the endoscope to allow reprocessing fluid to contact an internal channel of the endoscope, **characterised in that** the mounting is located within the reprocessing chamber and the connector is coterminous with the mounting, and wherein the endoscope carrier is rotatable relative to the reprocessing chamber such that reprocessing fluid emitted from the first reprocessing means contacts, at different angles, an endoscope positioned on or in the endoscope carrier.

2. The apparatus according to claim 1, in which the first reprocessing means is arranged to allow reprocessing fluid emitted therefrom to reprocess an exterior surface of the endoscope.

3. The apparatus according to any preceding claim, in which the mounting engages with a rotary shaft (4) disposed within the reprocessing chamber.

4. The apparatus according to any preceding claim, in which the endoscope carrier is rotated relative to the reprocessing chamber by reprocessing fluid emitted from the first reprocessing means.

5. The apparatus according to any preceding claim, in which the reprocessing chamber comprises an outlet (30) for drainage of reprocessing fluid, and the outlet is in fluid communication with a rotating drain valve (31).

6. The apparatus according to claim 5, in which the rotating drain valve comprises an indexing conduit rotatable to align with one of a plurality of exit conduits in fluid communication with the reprocessing fluid source and/or a gas extractor.

7. The apparatus according to any preceding claim, in which the endoscope carrier comprises a rack (7).

8. The apparatus according to any of claims 1 to 6, in which the endoscope carrier is a cartridge.

9. The apparatus according to claim 8, in which the cartridge comprises a second reprocessing means disposed on or in an internal surface of the cartridge and in fluid communication with the reprocessing fluid source.

10. The apparatus according to either of claim 8 or claim 9, in which the cartridge engages with the connector as defined in any of claims 1 to 9 to allow reprocessing fluid to contact a channel of an endoscope housed within the cartridge and/or to provide means for fluid communication between the reprocessing fluid source and the second reprocessing means.

11. The apparatus according to any one of the preceding claims in which the reprocessing fluid source in fluid communication with the connector is independent from or operable linked with the reprocessing fluid source in fluid communication with the first reprocessing means.

12. The apparatus according to any one of the preceding claims in which the endoscope carrier (6) is rotated relative to the reprocessing chamber through up to 360 degrees, for example up to 180 degrees.

13. The apparatus according to any one of the preceding claims wherein the reprocessing chamber (3)is circular.

14. The apparatus according to any one of the preceding claims wherein the first reprocessing means is rotatable within the reprocessing chamber.

15. A method for reprocessing an endoscope, comprising the step of subjecting the endoscope to a reprocessing cycle within an endoscope reprocessing apparatus as defined in any of the preceding claims.

## Patentansprüche

1. Endoskopaufbereitungsvorrichtung (1), umfassend eine Aufbereitungskammer (3), eine Halterung (5), an der ein Endoskopträger (6) angebracht ist, ein erstes Aufbereitungsmittel, das auf oder in einer Innenfläche der Aufbereitungskammer angeordnet ist, und eine mit dem ersten Aufbereitungsmittel in Fluidkommunikation befindliche Aufbereitungsfluidquelle (10A-C), einen mit der Aufbereitungsfluidquelle in Fluidkommunikation befindlichen Verbinder (52), wobei der Verbinder an einem Ende des Endoskops angebracht werden kann, um Aufbereitungsfluid mit einem inneren Kanal des Endoskops in Kontakt kommen zu lassen, **dadurch gekennzeichnet, dass** sich die Halterung in der Aufbereitungskammer befindet und der Verbinder mit der Halterung übereinstimmt, und wobei der Endoskopträger relativ zu der Aufbereitungskammer drehbar ist, so dass aus dem ersten Aufbereitungsmittel ausgegebenes Aufbereitungsfluid in verschiedenen Winkeln mit einem auf oder in dem Endoskopträger positionierten Endoskop in Kontakt kommt.

2. Vorrichtung nach Anspruch 1, bei der das erste Aufbereitungsmittel so angeordnet ist, dass von ihm ausgegebenes Aufbereitungsfluid eine Außenfläche des Endoskops aufbereiten kann.

3. Vorrichtung nach einem der vorhergehenden Ansprüche, bei der die Halterung mit einer in der Aufbereitungskammer angeordneten Drehwelle (4) in Eingriff ist.

4. Vorrichtung nach einem der vorhergehenden Ansprüche, bei der der Endoskopträger von Aufbereitungsfluid, das von dem ersten Aufbereitungsmittel ausgegeben wird, relativ zu der Aufbereitungskammer gedreht wird.

5. Vorrichtung nach einem der vorhergehenden Ansprüche, bei der die Aufbereitungskammer einen Auslass (30) zum Ablassen von Aufbereitungsfluid umfasst und der Auslass sich in Fluidkommunikation mit einem rotierenden Ablassventil (31) befindet.

6. Vorrichtung nach Anspruch 5, bei der das rotierende Ablassventil eine Teilbewegungsleitung umfasst, die gedreht werden kann, so dass sie auf eine von mehreren Ausgangsleitungen, die sich in Fluidkommunikation mit der Aufbereitungsfluidquelle und/oder einer Gasabziehvorrichtung befinden, ausgerichtet ist.

7. Vorrichtung nach einem der vorhergehenden Ansprüche, bei der der Endoskopträger ein Gestell (7) umfasst.

8. Vorrichtung nach einem der Ansprüche 1 bis 6, bei der der Endoskopträger eine Kassette ist.

9. Vorrichtung nach Anspruch 8, bei der die Kassette ein zweites Aufbereitungsmittel umfasst, das auf oder in einer Innenfläche der Kassette angeordnet ist und sich in Fluidkommunikation mit der Aufbereitungsfluidquelle befindet.

10. Vorrichtung nach Anspruch 8 oder Anspruch 9, bei der die Kassette mit dem Verbinder in Eingriff ist, wie in einem der Ansprüche 1 bis 9 definiert, damit Aufbereitungsfluid mit einem Kanal eines in der Kassette aufgenommenen Endoskops in Kontakt kommen kann und/oder um Mittel zur Fluidkommunikation zwischen der Aufbereitungsfluidquelle und dem zweiten Aufbereitungsmittel bereitzustellen.

11. Vorrichtung nach einem der vorhergehenden Ansprüche, bei der die mit dem Verbinder in Fluidkommunikation befindliche Aufbereitungsfluidquelle unabhängig von der mit dem ersten Aufbereitungsmittel in Fluidkommunikation befindlichen Aufbereitungsfluidquelle ist oder funktionell und mit ihr verbunden ist.

12. Vorrichtung nach einem der vorhergehenden Ansprüche, bei der der Endoskopträger (6) relativ zu der Aufbereitungskammer um bis zu 360 Grad, z.B. bis zu 180 Grad, gedreht wird.

13. Vorrichtung nach einem der vorhergehenden Ansprüche, bei der die Aufbereitungskammer (3) rund ist.

14. Vorrichtung nach einem der vorhergehenden Ansprüche, bei der das erste Aufbereitungsmittel in der Aufbereitungskammer drehbar ist.

15. Verfahren zur Aufbereitung eines Endoskops, umfassend den Schritt, bei dem das Endoskop einem Aufbereitungszyklus in einer Endoskopaufbereitungsvorrichtung nach einem der vorhergehenden Ansprüche unterzogen wird.

## Revendications

1. Appareil de retraitement d'endoscope (1) comprenant une chambre de retraitement (3), un support (5) auquel un dispositif porteur d'endoscope (6) est fixé, un premier moyen de retraitement disposé sur ou dans une surface interne de la chambre de retraitement, et une source de fluide de retraitement (10A-C) en communication fluide avec le premier moyen de retraitement, un connecteur (52) en communication fluide avec la source de fluide de retraitement, le connecteur fixable à une extrémité de l'endoscope pour permettre au fluide de retraitement d'entrer en contact avec un canal interne de l'endoscope, **caractérisé en ce que** le support est situé à l'intérieur de la chambre de retraitement et le connecteur est contigu au support, et où le dispositif porteur d'endoscope est pivotable par rapport à la chambre de retraitement de telle manière à ce que le fluide de retraitement émis à partir du premier moyen de retraitement entre en contact, à des angles différents, avec un endoscope positionné sur ou dans le dispositif porteur d'endoscope.

2. Appareil selon la revendication 1 dans lequel le premier moyen de retraitement est disposé pour permettre au fluide de retraitement émis à partir de celui-ci de retraiter une surface externe de l'endoscope.

3. Appareil selon une quelconque revendication précédente, dans lequel le support s'engage avec un arbre rotatif (4) disposé à l'intérieur de la chambre de retraitement.

4. Appareil selon une quelconque revendication précédente, dans lequel le dispositif porteur d'endoscope est pivoté par rapport à la chambre de retraitement par le fluide de retraitement émis à partir du premier moyen de retraitement.

5. Appareil selon une quelconque revendication précédente, dans lequel la chambre de retraitement comprend une sortie (30) pour le drainage du fluide de retraitement et la sortie est en communication fluide avec une soupape de drainage rotative (31).

6. Appareil selon la revendication 5, dans lequel la soupape de drainage rotative comprend un conduit d'indexage pivotable pour s'aligner sur l'un d'une pluralité de conduits de sortie en communication fluide avec la source de fluide de retraitement et/ou extracteur de gaz.

7. Appareil selon une quelconque revendication précédente, dans lequel le dispositif porteur d'endoscope comprend une crémaillère (7).

8. Appareil selon une quelconque des revendications de 1 à 6, dans lequel le dispositif porteur d'endoscope est une cartouche.

9. Appareil selon la revendication 8, dans lequel la cartouche comprend un second moyen de retraitement disposé sur ou dans une surface interne de la cartouche et en communication fluide avec la source de fluide de retraitement.

10. Appareil selon l'une ou l'autre de la revendication 8 ou revendication 9, dans lequel la cartouche s'engage avec le connecteur comme défini dans une quelconque des revendications de 1 à 9 pour permettre au fluide de retraitement d'entrer en contact avec un canal d'un endoscope logé à l'intérieur de la cartouche et/ou pour fournir des moyens de communication fluide entre la source de fluide de retraitement et le second moyen de retraitement.

11. Appareil selon une quelconque des revendications précédentes dans lequel la source de fluide de retraitement en communication fluide avec le connecteur est indépendante ou opérationnelle et liée à la source de fluide de retraitement en communication fluide avec le premier moyen de retraitement.

12. Appareil selon une quelconque des revendications précédentes dans lequel le dispositif porteur d'endoscope (6) est pivoté par rapport à la chambre de retraitement jusqu'à 360 degrés, par exemple jusqu'à 180 degrés.

13. Appareil selon une quelconque des revendications précédentes où la chambre de retraitement (3) est circulaire.

14. Appareil selon une quelconque des revendications précédentes où le premier moyen de retraitement est pivotable à l'intérieur de la chambre de retraitement.

15. Méthode pour retraiter un endoscope comprenant l'étape consistant à soumettre l'endoscope à un cycle de retraitement à l'intérieur d'un appareil de retraitement d'endoscope comme défini dans une quelconque des revendications précédentes.
